# EUROPEAN PATENT SPECIFICATION

(11) **EP 4 458 263 B1**
(45) Date of publication and mention of the grant of the patent: **18.03.2026**
(21) Application number: 24173782.4
(22) Date of filing: 02.05.2024
(51) Int. Cl.: A61B 5/0538, A61B 5/06, A61B 5/287, A61B 5/00

(54) **SINGLE SENSOR FOR PHYSIOLOGIC SIGNAL MEASUREMENT WITH POSITION AND TISSUE PROXIMITY INDICATION**
EINZELSENSOR ZUR MESSUNG PHYSIOLOGISCHER SIGNALE MIT POSITIONS- UND GEWEBENÄHERUNGSANZEIGE
CAPTEUR UNIQUE POUR LA MESURE DE SIGNAUX PHYSIOLOGIQUES AVEC INDICATION DE POSITION ET DE PROXIMITÉ DE TISSU

(30) Priority: 03.05.2023 US 202318311606
(43) Date of publication of application: 06.11.2024
(73) Proprietor: Biosense Webster (Israel) Ltd., 2066717 Yokneam (IL)
(72) Inventor: BOTZER, Lior, 2066717 Yokneam (IL); SUAREZ, Paul, Irvine, 92618 (US); HIGHSMITH, Debby, Irvine, 92618 (US); AMEFIA, Kokou Anani Mawuena, Irvine, 92618 (US)
(74) Representative: Carpmaels & Ransford LLP

(56) References cited:
- US-A1- 2012 143 293
- US-A1- 2015 073 515
- US-A1- 2015 320 974
- US-A1- 2018 000 540
- US-A1- 2018 243 536

## Description

### BACKGROUND

Catheters may include physiologic signal sensing electrodes (e.g., ECG electrodes) for sensing a parameter of interest and magnetic position sensors to accurately track location of the catheter. The magnetic position sensor is a coil through which a magnetic field may be generated. The coil is dedicated to sensing the position of the catheter in the body. Presently, such dedicated coils are only used for magnetic position sensing.

Catheters that do not include magnetic position sensors use Active Current Location (ACL) components and systems, to track the location of the catheter. ACL relies upon injection of a small electrical current from an electrode on the catheter inside the body so that patch electrodes outside the body can be used to triangulate the location of the catheter electrode from measurements of the resulting impedance of the electrical current through the body. Example ACL systems include those detailed in US Patent No. 7,756,576. 7,869,865, 7,848,787, and 8,456,182, and are typically associated with CARTO^{®} systems and procedures, from Biosense Webster, the owner of this application. However, when compared to magnetic position sensors, ACL systems drive current and calculate positions based on impedance, and accordingly, are much less accurate than magnetic position sensors.

### SUMMARY OF THE DISCLOSURE

The present disclosed subject matter provides a multi-functional sensor unit that can be used to: (1) measure physiologic electrical signals generated by tissues (e.g., ECG signals); (2) provide indication of tissue proximity to the sensor; and (3) sense one or more magnetic fields so as to provide a location of the multi-functional sensor unit relative to the one or more magnetic fields. The integrated multi-functional sensor unit, typically used on a catheter is configured with an outer coil extending along the catheter. The coil is formed of wire windings, with at least two of the windings uninsulated or exposed. These exposed windings allow the multi-functional sensor unit to sense environmental impedance and physiologic, sent as electrical impulses (signals) from the heart (e.g., heart wall) during contraction. The coil has an inductive impedance that is determined by the coil geometry and a secondary impedance determined by the environment to which the exposed windings are exposed. The secondary impedance may include a resistive component, a capacitive component, an inductive component, or any combination thereof. The secondary impedance may be a complex impedance. The overall impedance of the coil therefore changes with the environment, and the change in impedance can be detected based on electrical signals (e.g. current and/or voltage signals) output from the coil which are induced by a known magnetic flux from a generated magnetic field acting on the coil. The change in coil impedance can provide an indication of proximity to tissue because tissue has a different impedance than blood, for instance tissue has a resistive impedance component that is less than blood. Therefore the coil is more inductive when the exposed portions are away from tissue and the coil has a lower resistance when the exposed portions are moved close to tissue. The exposed windings can measure physiologic signals (e.g. ECG signals) when in contact with biological tissue. In some examples, the inductance of the coil can allow the coil to function as a position sensor.

The coil can have many configurations to achieve the aforementioned functionality. The coil can include a coaxial wire having a core conductive member, an inner insulation over the core conductive member, an outer conductive member over the inner insulation, and an outer insulation over a portion of the outer conductive member. The sensor can have insulated coil windings interleaved with exposed coil windings. The insulated and exposed coil windings can form the aforementioned coil (i.e. a single coil), or the sensor can include a second, insulated coil in addition to the coil with at least two exposed winding portions. The coil can be wound such that uninsulated portions of the coil touch when the coil is bent, thereby causing a short between coil windings and changing the impedance of the coil so that the change in impedance can be measured to indicate curvature of the coil. The coil can be integrated with a medical probe such as a catheter or a guide wire. Coil windings can be stacked radially from a central axis of the coil with insulated coil portions overlapping uninsulated coil portions. Compatible coil configurations are combinable as understood by a person skilled in the pertinent art.

### BRIEF DESCRIPTION OF THE DRAWINGS

The present disclosure will be more fully understood from the following detailed description of embodiments thereof, taken together with the drawings, in which:
Fig. 1 is a schematic illustration of an electroanatomical mapping system for cardiac mapping, which includes a catheter on which the disclosed sensor units are deployed;
Fig. 2 is an illustration of a first exemplary sensor unit disposed on a portion of a catheter illustrating features of the multi-functional sensor unit of Fig. 1, in accordance with an example of the present disclosure;
Fig. 3 is a flow diagram of example processes for acquiring data from the disclosed sensor units to produce various outputs;
Fig. 4 is an illustration of a second exemplary sensor unit disposed on a portion of a catheter and illustrating features of the multi-functional sensor unit of Fig. 1, in accordance with an example of the present disclosure;
Figs. 5A and 5B are illustrations of an example catheter having a third exemplary sensor unit and illustrating features of the multi-functional sensor unit of Fig. 1, in accordance with an example of the present disclosure;
Fig. 6A is an illustration of a fourth exemplary sensor unit illustrating features of the multi-functional sensor unit of Fig. 1, in accordance with an example of the present disclosure;
Figs. 6B and 6C are illustrations of cross-sections of a coaxial wire of the fourth exemplary sensor unit as indicated in Fig. 6A;
Figs. 7A and 7B are illustrations of a fifth exemplary sensor unit illustrating features of the multi-functional sensor unit of Fig. 1, in accordance with an example of the present disclosure;
Fig. 8A is an illustration of a sixth exemplary sensor unit illustrating features of the multi-functional sensor unit of Fig. 1, in accordance with an example of the present disclosure;
Fig. 8B is an illustration of a seventh exemplary sensor unit illustrating features of the multi-functional sensor unit of Fig. 1, in accordance with an example of the present disclosure;
Fig. 9 is an illustration of a guide wire including an eighth exemplary sensor unit, in accordance with an example of the present disclosure;
Fig. 10 s an illustration of a ninth exemplary sensor unit illustrating features of the multi-functional sensor unit of Fig. 1, in accordance with an example of the present disclosure;
Fig. 11 is an illustration of a tenth exemplary sensor unit illustrating features of the multi-functional sensor unit of Fig. 1, in accordance with an example of the present disclosure;
Fig. 12 is an illustration of an eleventh exemplary sensor unit illustrating features of the multi-functional sensor unit of Fig. 1, in accordance with an example of the present disclosure; and
Figs. 13A and 13B are illustrations of an impedance model of the multi-functional sensor unit of Fig. 1, in accordance with an example of the present disclosure.

### DETAILED DESCRIPTION OF EXAMPLES

### OVERVIEW

The following detailed description should be read with reference to the drawings, in which like elements in different drawings are identically numbered. The drawings, which are not necessarily to scale, depict selected embodiments and are not intended to limit the scope of the invention, which is defined by the claims. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±10% of the recited value, e.g., "about 90%" may refer to the range of values from 81% to 99%. In addition, as used herein, the terms "patient," "host," "user," and "subject" refer to any human or animal subject and are not intended to limit the systems or methods to human use, although use of the subject invention in a human patient represents a preferred embodiment.

As used herein, the term "multi-functional sensor" or "sensor unit" indicates a device with two coils capable of providing at least three functions (as further discussed and illustrated elsewhere in this document) when connected to an appropriate electrophysiologic system such as that of CARTO^{®} (manufactured by Biosense Webster Inc.,). Such "multi-functional sensor" or "sensor unit" is capable of:
(1) measuring or recording physiologic electrical signals generated or propagated by biological tissues (e.g., ECG signals);
(2) indicating tissue proximity or tissue contact to the sensor; and
(3) sensing one or more magnetic fields so as to provide a location of the multi-functional sensor unit relative to the one or more referential magnetic fields. It is noted that all three functions are not required to be enabled concurrently and can be used independently.

The present disclosed subject matter provides sensor units or sensors, for example, operational with catheters, such as a focal catheter or other catheter, which is used, for example, in procedures such as electroanatomical mapping of a body organ, such as the heart. The electroanatomical mapping may be part of an electrophysiology system such as CARTO^{®} (manufactured by Biosense Webster Inc.,) which can be used for electrophysiological procedures. The integrated sensor units are positioned on the catheter, at various known distances from each other, and typically the same distance from each other. Each of the sensor units includes an inner coil, inside of the catheter, which is electrically insulated, for example, by the catheter, from contacting tissue, blood or other bodily fluids, which, when in the body, and at least a partially uninsulated outer coil, outside of the catheter, which when in the body is directly exposed to and potentially in contact with tissue, blood and other bodily fluids.

The insulated inner coil functions, for example, as a magnetic position sensor. The inner coil is such that in the presence of a magnetic field, the inner coil outputs signals, which are usable to ascertain the location and/or orientation of the catheter on which the sensor units are disposed.

The uninsulated outer coil performs a plurality of functions. A first function includes a physiologic signal sensing or recording electrode, where the outer coil exchanges signals with tissue, such as by acquiring physiologic signals (e.g., electrocardiogram (ECG) from cardiac tissue). In the case of cardiac tissue, the physiologic signals (e.g. ECG signals) are produced by electrical currents in the tissues of the heart which cause contractions in the heart walls, and create different potentials through the body. The outer coil outputs these physiologic signals (e.g., ECG signals) to a processor or computer, which, for example, uses these signals in creating a cardiac map, for example, as part of a CARTO^{®} procedure, or performs other analysis thereof.

A second function includes operation as a conductivity sensor, which is affected by impedance, for example, electrical impedance or environmental impedance, as a generated electric field passes through tissue/blood, resulting in changes in the impedance, based on the various tissue and/or fluid that the electric field passes through. The detected (sensed) conductivity values of the electric flux are output by the outer coil as complex voltages (and/or current) to a processor or computer. The output voltages correspond to impedance in tissues and/or fluids and can further be used to provide indication on proximity to the tissue, and are indicative of various aspects of tissues, such as of tissue types, fluid types, e.g., blood or other bodily fluids, in the body location where the catheter is operating, as analyzed by the processor and/or computer. Additionally, as the outer uninsulated coil operates with the aforementioned generated referential magnetic field, it may also function as a magnetic position sensor, similar to the magnetic position sensor operation of the inner coil.

Since the inner and outer coils operate with a generated magnetic field, for example, from magnetic field generators proximate (e.g., underneath or in the vicinity of the subject in which the catheter is operating) to the catheter with the sensor units, the inner and outer coils typically do not drive current, for to do so would interfere with the generated magnetic field that the inner and outer coils are used to detect. The plurality of functions performed by the outer uninsulated coil may be performed contemporaneously, and in some cases, simultaneously.

Additionally, as the outer coil performs multiple functions, it replaces individual sensors previously used for each of the functions, therefore, reducing complexity of the catheter and system associated therewith.

The present disclosure also processes signals received from the above-described inner coil and outer coil, a plurality of outputs, which are used by a processor and/or computer to construct an electroanatomical map. These outputs include a plurality of first outputs from the outer coil, which indicate physiologic signals (e.g., the electrical activity of the tissue, ECG, etc.), and a plurality of second outputs, also from the outer coil, which indicate the respective induced voltage differences across the outer coil corresponding to current that is leaking between individual windings in the outer exposed coil. This leaked current is related to tissue/material surrounding the outer coils.

There are also a plurality of third outputs, at least from the inner coil, which indicate the proximity to certain tissue or the position of each of the sensor units, and accordingly, the catheter. In some instances, the outer coil may also function to produce the aforementioned third outputs for position of the catheter and/or the sensor units thereon.

### APPARATUS DESCRIPTION

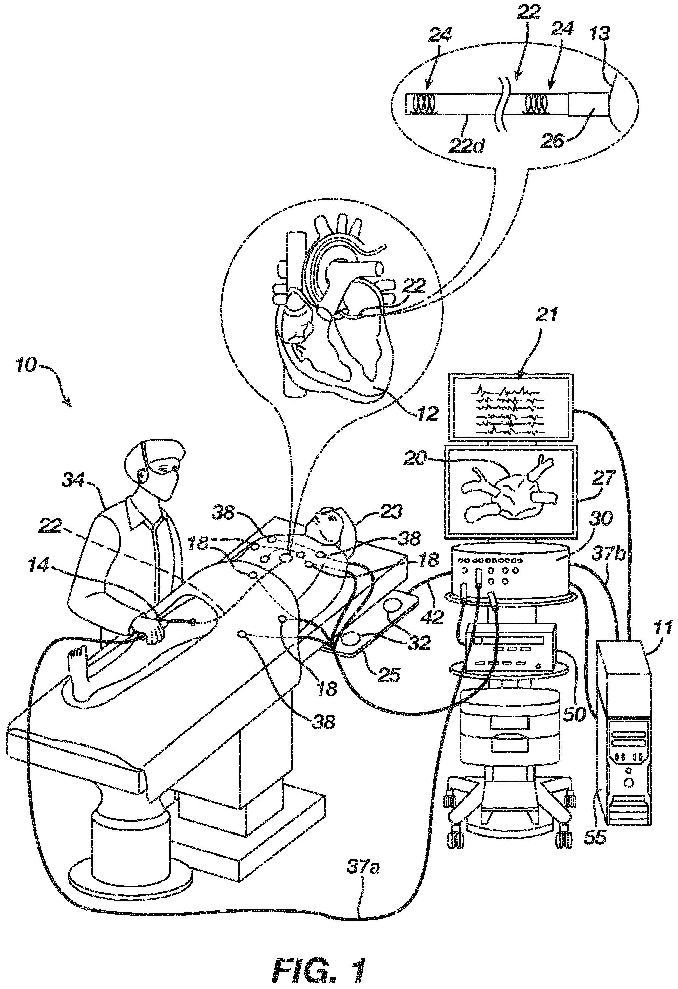

Reference is made to FIG. 1 showing an example catheter-based electrophysiology mapping and ablation system 10. System 10 includes multiple catheters, which are percutaneously inserted by physician 34 through the patient's vascular system into a chamber or vascular structure of a heart 12. Typically, a delivery sheath 14, one or more catheters, represented by the catheter 22 is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating.

An example catheter 22 such as a focal catheter, is used to perform an electroanatomical mapping and/or ablation of the heart 12 of a subject 23. During the mapping procedure, the distal end 22d of the catheter 22, which comprises one or more sensor units or sensors 24, is inserted into heart 12. For IEGM procedures, the physician 34 brings a distal tip 26 of the catheter 22 into contact with the heart wall 13 for sensing a target site in the heart 12. For ablation, the physician 34 similarly brings the distal end 26 of the catheter 22 to a target site 13 for ablating. The same catheter 22 may be used for sensing and ablation, and/or multiple catheters can be used to sense and ablate with some of the catheters being specialized for sensing or ablation.

The illustrated focal catheter 22 includes sensor units 24, which can have various features as disclosed in the exemplary sensor units described in detail below. The focal catheter 22 is one of example of a catheter which can be modified to include sensor units 24. A multi-electrode catheter having an end effector with multiple electrodes for sensing and/or ablation can also be modified to include the sensor units 24 on the shaft of the catheter adjacent the end effector, or on the end effector. For instance, an end effector can include a sensor unit 24 coupled to a spine of a basket, ray, planar, lasso, or other such end effector structure including one or more spines.

Each sensor unit 24, for example, functions as a physiologic sensing or recording electrode, to detect physiologic electrical signals (e.g., ECG), as well as functioning as inductance sensors, to detect changes in magnetic flux, from the generated magnetic fields (generated by magnetic-field generators 32, i.e., coils or magnetic radiators, located underneath subject 23 or otherwise in the vicinity of the subject 23), and output these detected changes as voltages, e.g., voltage changes. The detected magnetic field (magnetic flux) changes, as voltages, are input into the processor (not shown) in the workstation 55, which is programmed, for example, to analyze tissue impedance (Tissue Proximity Indicator (TPI)), for example, based the received voltage changes. These received voltages are used to deduce information about tissues and/or fluids proximate to the catheter 22 and/or each sensor unit 24, as well as to detect the position of the catheter 22 and/or sensor unit 24 on the catheter, in the body, for example, the heart 12.

The integrated sensor units 24 communicate with the processor of the workstation 55. The processor, for example, is programmed to analyze the various signals captured by the multi-functional sensor unit 24. The received signals and magnetic field data are transmitted from the multi-functional sensor unit 24 to the processor, for example, over one or more lines 37a, 37b.

Typically, the processor comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

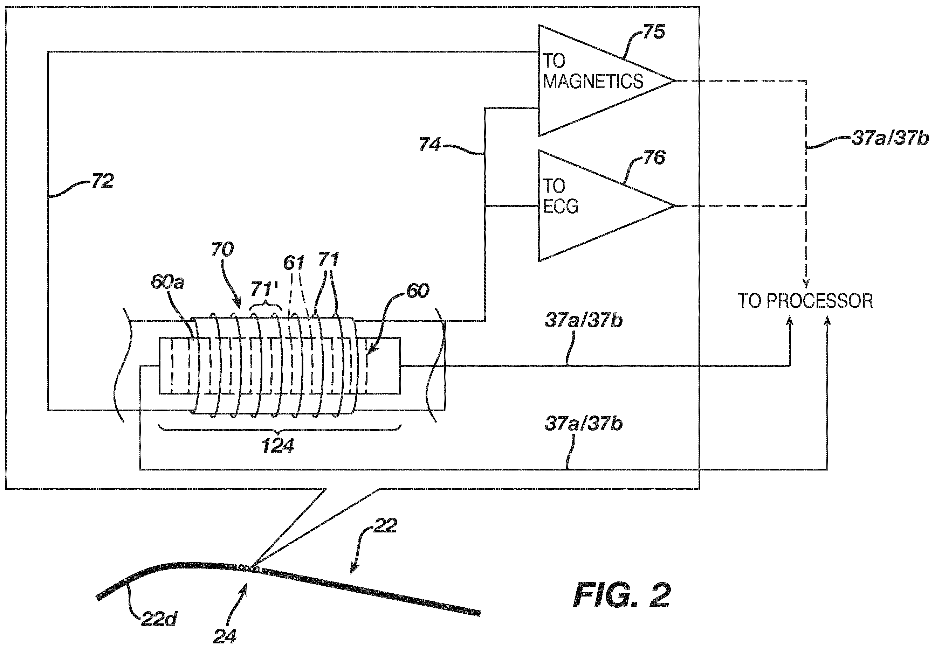

A signal generator, for example, in the patient interface unit (PIU) 30, typically causes the generators 32 to generate the magnetic field by supplying an alternating current (e.g., over the line 42) to the generators 32. The generated magnetic field induces voltage differences across the inner 60 and outer 70 coils (Fig. 2) (the inner 60 and outer 70 coils also known as conducting elements, inner and outer, respectively) detailed below, of the multi-functional sensor unit 24. For example, the induced voltage differences (output by the inner 60 and outer 70 coils, respectively) are received by the PIU 30 and then the processor in the workstation 55 (via the lines 37a, 37b), and, based on the induced voltages, the processor ascertains the position of at least the inner coil 60, and in some cases, also the outer coil 70, as well as tissue impedance (also known as environmental impedance) measurements (tissue proximity indicators (TPI)) from the outer coil 70 (for the location in the body of the catheter 22).

For example, the processor may be programmed to compare the voltages, representative of various tissue and/or fluid impedances, to voltages in a database(s), look up table (LUT), other stored values, or references, or the like, to determine the tissue and/or fluid type. The tissue and/or fluid types may be displayed on the monitor 27.

The processor is typically also programmed to construct an electroanatomical map 20 of the heart 12 (the body location where the catheter 22 operates in this example), based on the physiologic signals (e.g., ECG signals which indicate the electrical activity of the intracardiac tissue) and the voltages received from the helical conducting elements (which indicate the respective locations of the sources of the ECG signals). Such an anatomical map overlaid with physiologic signals (e.g., ECG signals) ("electroanatomical map") map 20 may be displayed on the monitor 27 for viewing by physician 34, and/or stored for later analysis.

The integrated sensor units 24 may be arranged at intervals of various distances apart from each other, for example, approximately 1 mm to 4 mm. For example, the distances between the sensor units 24 are the same, and the locations of the sensor units 24 on the catheter 22 is known.

A recorder 11 displays electrograms 21 captured with body surface ECG electrodes 18 and intracardiac electrograms (IEGM) captured with sensor units 24 of catheter 22. The recorder 11 may include pacing capability for pacing the heart rhythm and/or may be electrically connected to a standalone pacer.

System 10 may include an ablation energy generator 50 that is adapted to conduct ablative energy to one or more of electrodes at the distal tip 26 of the catheter 22 or a distal tip of another example catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

The patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (4) displaying on display device 27 sites of interest such as places where ablation energy has been applied. One commercial product embodying elements of the system 10 is available as the CARTO^{™} 3 System, available from Biosense Webster, Inc., 31 Technology Drive, Irvine, CA 92618.

Turning also to Fig. 2, where a section of the catheter 22 is shown with a first exemplary sensor unit 124, which illustrates example features of the multi-functional sensor unit 24 illustrated in Fig. 1. The first exemplary sensor unit 124 is formed of an inner conducting element, for example, a coil 60 (also known as the inner coil) of wire(s) 61, inside of the catheter 22, and, an outer conducting element, for example, a coil 70 (also known as the outer coil), outside of the catheter 22, also formed, for example, of a coiled electrically conductive wire or wires 71 (or windings or helical windings), with at least a portion of the wires 71' of the outer coil 70 being uninsulated and exposed. For example, the uninsulated or exposed portion of the outer coil 70 includes at least two windings of the outer coil 70. Accordingly, voltage output of the inner coil 60, which is, for example, fully insulated, is affected primarily by the flux of the magnetic field through the core of the inner coil 60, wherein the flux of the magnetic field is primarily based on magnetic fields as generated by the generators 32. The voltage output of the outer coil 70 is affected by magnetic flux through the core of the outer coil 70, wherein the magnetic flux is primarily based on magnetic fields provided by the magnetic radiators 32 of the location pad 25 and is similar to the flux of the magnetic field through the core of the inner coil 60 where the outer and inner coils 70, 60 are coaxial. The voltage output of the outer coil 70 is also affected by changes in secondary impedance between uninsulated windings 71' of the outer coil 70 due to direct physical contact of the uninsulated windings 71' with the external environment (e.g., blood or tissue).

Because the inner coil 60 is insulated, the impedance of the inner coil 60 is primarily inductive and known based on geometry of the inner coil 60. Magnetic flux varies in space in a known way based on externally applied magnetic fields generated by generator coils 32. The magnetic flux through the core of the inner coil 60 causes the inner coil 60 to provide an electrical signal to the workstation 55. The electrical signal is a function of the magnetic flux and the impedance of the inner coil 60. Because the impedance of the inner coil 60 is known and the magnetic flux is a known function of position, position of the inner coil 60 can be determined based on electrical signals from the inner coil 60. The electrical signal can be used to determine a location of the first exemplary sensor unit 124 using magnetic based position sensing technology such described, as non-limiting examples, in U.S. Patent Nos. 5,391,199; 5,443,489; 5,558,091; 6,172,499; 6,239,724; 6,332,089; 6,484,118; 6,618,612; 6,690,963; 6,788,967; 6,892,091. Because the inner coil 60 and the outer coil 70 are coaxial, the magnetic flux through the inner coil 60 is approximately the same as the magnetic flux through the outer coil 70, which is related to the electrical signal from the inner coil 60. The impedance of the outer coil 70 can be calculated, by the workstation 55 based on an output signal (e.g. voltage) of the outer coil 70 and the magnetic flux through the core of the outer coil 70 (i.e. based on the electrical signal from the inner coil 60). The impedance of the outer coil includes inductance based on the geometry of the coil windings and secondary impedance based on conductivity through the environment in the vicinity of exposed portions of uninsulated windings 71'. Complex impedance of the outer coil is therefore based on the geometry of the coil and location of exposed portions (which is known) and impedance of the environment between the exposed portions. Therefore, changes in electrical conductivity of the environment in the vicinity of the exposed portions of the uninsulated windings 71' of the outer coil 70 can be detected based on output voltages of the inner coil 60 and the outer coil 70.

The inner coil 60 windings 61 are electrically insulated, for example, by the catheter 22 itself, or other insulation material 60a inside of the catheter 22. The inner coil 60 includes, for example, a dedicated voltage channel, which includes the line 37a, 37b, so that the induced voltage differences caused by the generated magnetic field (e.g., the magnetic field generates a potential on the inner coil 60), are relayed to the processor (not shown) in the workstation 55. The processor analyzes the received voltages (potentials) and ascertains, or otherwise determines, the position of the inner coil 60 within the body, for example, within the heart 12.

The outer coil 70, includes, for example, windings 71 positioned along the outer surface of the catheter 22. The windings 71 include at least two uninsulated wire portions 71'. The outer coil 70 and inner coil 60 are, for example, arranged so as to be coaxial and/or concentric with respect to each other. For example, the outer coil 70 extends over (and along) at least a portion of the inner coil 60 and/or is shifted with known bias, so as to extend within the span of the inner coil 60 (e.g., the inner coil 60 typically extends longitudinally beyond the outer coil 70). The outer coil 70 may be wound at a first helix angle for the outer coil winding (wire(s) 71) while the inner coil 60 may be wound (wire(s) 61) at a second helix angle for the inner coil winding being different from the first helix winding angle. The two known but different first and second helical winding angles will allow for the inner coil 60 and outer coil 70 to act as a dual-axis magnetic location sensor.

The outer coil 70 includes a dedicated voltage channel (for measuring potential), which includes sublines 72, 74 for signals representative of received magnetics by operational amplifier (op-amp) 75, and signals representative of received physiologic signals (e.g., ECG signals), by op-amp 76, which communicate with the processor in the workstation 55 via the lines 37a, 37b. The outer coil 70, by virtue of the wire(s) 71 which form it, include a portion 71' being uninsulated and otherwise exposed, contacts body tissues and/or fluids, such as blood, and the like.

The outer coil 70, is formed, for example, from a single coil, or multiple coils joined to form the outer coil 70, with an insulated wire(s). The wire(s) 71 are wound in a series of helical windings or turns around the catheter 22. A portion of the insultation is removed from the wire(s), for example, mechanically by a laser, to expose portions of the wire(s). The exposure of the wire portions is made in a manner that does not lead to contact between the windings so that secondary impedance between uninsulated windings 71' can be sufficiently high in certain environmental conditions to allow the inductive portion of the impedance of the outer coil 70 to be significant. Accordingly, a partially exposed outer coil 70 may function as a position sensor, similar to that of the inner coil 60.

The outer coil 70, for example, via its at least two, and typically, two or more uninsulated or exposed windings, performs a plurality of functions. One function of the outer coil 70 is as an electrode, where the outer coil 70 exchanges signals with tissue, such as by acquiring physiologic electrical signals (e.g., ECG) from tissue or to drive electrical signals from the outer coil 70 into the tissues such as for pacing when exposed portions of the uninsulated windings 71' are in contact with tissue. Another function is an environmental conductivity sensor which includes a magnetic field sensor unit (sensor) 124 or flux sensor, which generates an output voltage based on the magnetic field generated (by magnetic field generators 32 disposed at referential locations under or around the patient) as received by the outer coil 70, inductive impedance of the outer coil 70 based on geometry of the outer coil, and secondary impedance of the outer coil 70 based on conductivity of tissues and/or fluids between exposed portions of uninsulated windings 71' of the outer coil 70. The tissue conductivity/impedance values, and accordingly, the output voltages, are different for each type of tissue and/or fluid, and are, for example, used by the processor of workstation 55 to determine one or more of tissue types, tissue boundaries, locations of certain tissue, and/or fluids, and the like between the exposed portions of uninsulated windings 71'.

The outer coil 70 is such that different frequency bands may be used for analyzing position and tissue impedances (e.g., parameters) through the outer coil 70 and the lines 72, 74 extending from the wires 71 of the outer coil 70. For example, ECG may occupy DC to 1 kHz while magnetic related voltage, and in some cases, also magnetic position sensing, may use, for example, approximately 2 kHz to 8 kHz. Since the frequency spectra for ECG (electrical activity sensing) and position sensing is different, the two signals do not overlap, and therefore, may be concurrently transmitted by the same wire.

The outer coil 70 may also function as a magnetic position sensor, identical or similar to that of inner coil 60, when the secondary impedance of the outer coil 70 is sufficiently high and/or known. Based on the detected outer coil 70, the position of the outer coil 70 and/or the first exemplary sensor unit 124 in the body, for example, the heart 12, is determined. When the outer coil 70 is used as a position sensor (as detailed below), the inner coil 60 serves as a reference.

Alternately, the inner coil 60 need not be present, provided that there is a known physical relationship between an external leaky coil (e.g. outer coil 70) and an external sensor, such as the first exemplary sensor unit 124. "Leaky" in this context is that the coil has an impedance that can be modeled as a non-ideal inductor that has an ideal inductive impedance in addition to a significant secondary impedance.

Changes in impedance of the environment is detected between two or more exposed portions of two or more windings (wire portion 71') of the outer coil 70. Alternatively, the outer coil 70 may include only one winding with one or more exposed portions, and the catheter 22 can further include a secondary exposed electrode or sensor (e.g. ACL location sensor) disposed approximate the outer coil 70 configured such that a change in impedance can be measured between the exposed winding and the secondary exposed electrode or sensor. In this example, the one exposed winding is usable for physiologic signal sensing (e.g., ECG) when in contact with tissue, and changed in environmental impedance can be detected based on a change in impedance between the exposed winding and the secondary exposed electrode or sensor. A disadvantage of this configuration is that it may be required to drive a current through the outer coil 70 and/or secondary electrode/sensor.

The disclosed outer coil 70 is designed with two or more uninsulated and exposed windings, so that the outer coil 70 can operate absent a secondary electrode or sensor (e.g. absent ACL location sensing systems), and, as such current does not need to be driven through the outer coil 70 (or the inner coil 60), which would have the potential to interfere with the magnetic fields or the physiologic signals (e.g., ECG signals) from the generators 30 and comes with a need for specific hardware that generates and measures the current. Moreover, by having at least two uninsulated and exposed windings of the outer coil 70, position calculations are not based on impedance, as is the case with ACL systems.

The windings of the outer coil 70, and at least the uninsulated or exposed windings (wire portion 71) are, for example, spaced from each other at least at a minimum distance, to avoid interference, including electrical interference, which is a potential cause of short circuiting. This minimum distance is, for example, at least approximately 0.5 mm. The distance between the windings affects sensitivity. For example, a larger distance or spacing between the individual windings renders the outer coil 70 less sensitive, when compared to the outer coil 70 having a shorter separation distance between windings. The number of windings (turns) of the coil may also affect sensitivity. Also, the material of the outer coil 70 affects the sensitivity, as well as the distance between the windings.

The leakage current between the windings of the outer coil 70 is affected by the gradient voltage on the outer coil 70. Different winding materials will generate different voltages and current leakage. The higher the voltage gradient on the outer coil 70, the larger the sensitivity to environmental/tissue related impedance. Materials for the wire coils 70 include, for example, medical grade Stainless Steel, and platinum wire. The wire can be also coated at intermediate locations between one or more windings, to achieve better/tailored sensitivity in specific areas of the exposed wire.

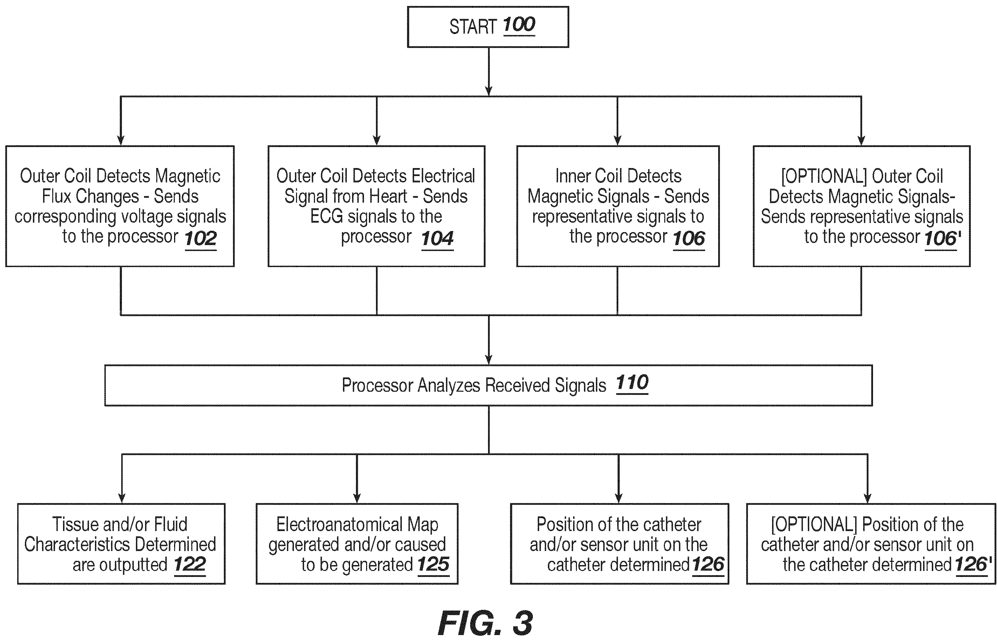

Fig. 3 is a flow diagram of example processes performed by the processor, based on signals received from the outer coil 70, and in some cases, also the inner coil 60. The processes are, for example, performed automatically and in real time, and may include manual subprocesses. The process may be performed as long as desired.

The process begins at a START block 100, where, for example, the catheter 22 is deployed in the subject 23, for example, in the heart 12. Additionally, a magnetic field is being generated by the generators 32, as controlled via the workstation 55.

At block 102, voltage signals corresponding to the changes in magnetic flux through the first exemplary sensor unit 124 of the catheter 22 and conductivity of environment as detected by the uninsulated and exposed windings (wire portion 71') of the outer coil 70, are sent (outputted) to the processor 32. At block 110, the processor of the workstation 55 analyzes these signals, for example, based the received voltage changes from the outer coil 70. These received voltages changes are used to deduce information about tissues and/or fluids proximate to the catheter 22 and/or each sensor unit 124, as well as to detect the position of the catheter 22 and/or sensor unit 124 on the catheter, in the body, for example, the heart 12. At block 122, the processor outputs tissue and/or fluid characteristics, such as tissue impedance value or tissue or fluid types, tissue and/or fluid boundaries, and the like.

At block 104, physiological signals (e.g., ECG signals) are received, through portions of exposed windings 71', by the outer coil 70 and sent (outputted) to the processor. The processor analyzes these signals, at block 110, and, at block 125, generates, or otherwise produces, or causes the production of an electro anatomical map of the heart 12.

At block 106 the inner coil 60 detects the generated magnetic field, and sends corresponding signal to the processor. The processor analyzes these received signals at block 110, and at block 126, determines a position of the catheter 22 and/or the multi-functional sensor unit 24 thereon.

In an optional process at block 106' the outer coil 70 detects the generated magnetic field, and sends corresponding signal to the processor. The processor analyzes these received signals at block 110, and at block 126', determines a position of the catheter 22 and/or the multi-functional sensor unit 24 thereon.

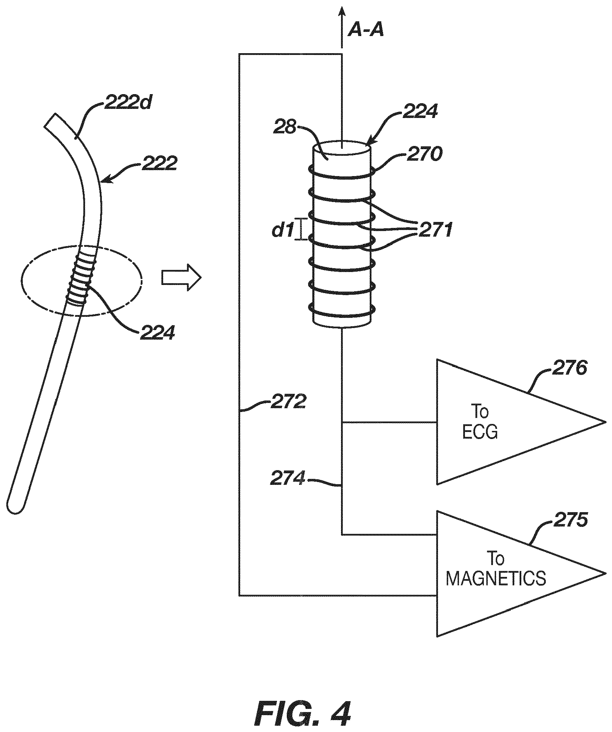

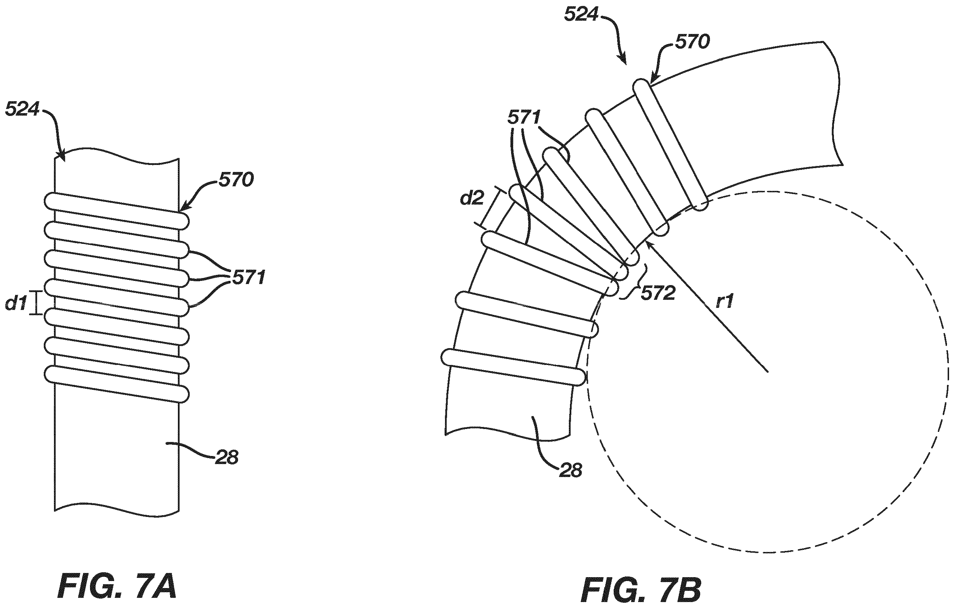

Fig. 4 is an illustration of a second exemplary sensor unit 224 disposed on a portion of a catheter 222 near a distal end 222d of the catheter 222 and illustrating features of the multi-functional sensor unit 24 of Fig. 1. The second exemplary sensor unit 224 includes a single coil 270 having at least two exposed, uninsulated portions, and may be entirely uninsulated. The coil 270 can include windings 271 spaced apart by a distance d1. The distance d1 can be sufficiently large so that adjacent uninsulated windings 271 do not make contact with each other. The distance d1 may also be configured so that adjacent windings short when the second exemplary sensor unit 224 is bent such as disclosed in greater detail in relation to Figs. 7A and 7B.

The coil 270 is electrically coupled to sublines 272, 274 to deliver electrical signals from the coil 270 to an ECG op-amp 276 and a magnetics op-amp 275. The ECG op-amp 276 is configured to communicate with the processor in the workstation 55 (Fig. 1) to transmit physiologic signals (e.g., ECG signals) from tissue 13 to exposed portions of the coil 270 pressed to the tissue 13. The magnetics op-amp 275 is configured to transmit, to the processor in the workstation 55 (Fig. 1), electrical signals indicating voltage across the coil 270 due to a magnetic field generated by the magnetic field generators 32. The coil 270 may also function as a magnetic position sensor, identical or similar to that of inner coil 60 of the first exemplary sensor 124 illustrated in Fig. 2. For instance, if the secondary impedance between uninsulated wire portions is sufficiently high so that the coil impedance is approximately equal to the inductive portion of the coil impedance, the electrical signal induced by the magnetic field generated by the magnetic field generators 32 can provide information as to the location of the coil 270 within the generated field, and therefore a position within the patient 23. As another alternative, the workstation 55 may be able to distinguish the inductive portion of the impedance of the coil 270 from the resistive (and/or capacitive) portion of the impedance of the coil 270, even when the resistive (and/or capacitive) portion of the impedance is significant. For instance, generators 32 may provide flux at multiple frequencies, and the frequency response of the electrical signal output from the coil 270 may be used to determine both impedance and location of the coil 270.

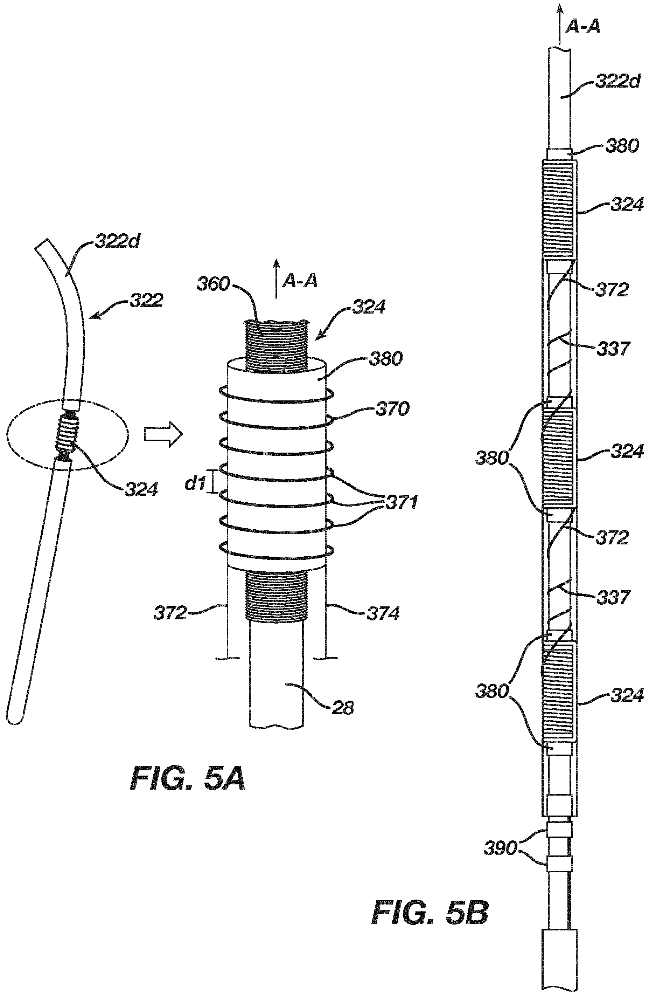

Figs. 5A and 5B are illustrations of an example catheter 322 having a third multi-functional exemplary sensor unit 324 and illustrating features of the multi-functional sensor unit 24 of Fig. 1. The third exemplary sensor unit 324 includes an inner coil 360 and an outer coil 370 configured similarly to the inner coil 60 and the outer coil 70 of the first exemplary sensor unit 124 illustrated in Fig. 2. The outer coil 370 can include at least two exposed uninsulated portions. The outer coil 370 can be configured to output signals based in a change in magnetic flux through the third exemplary sensor unit 324. The change in magnetic flux can provide an electrical signal indicative of impedance of the outer coil 370, which can be used to determine proximity to tissue as disclosed in greater detail in relation to Figs. 1, 2, 13A, and 13B. The uninsulated portions of the outer coil 370 can be configured to contact tissue and output physiologic signals (e.g, ECG signals) measured through tissue. The outer coil 370 may also function as a position sensor similar to the coil 270 of the second exemplary sensor unit 242 illustrated in Fig. 4 or the outer coil 70 of the first exemplary sensor unit 124 illustrated in Fig. 2. The inner coil 360 can function similarly to the inner coil 60 of the first exemplary sensor unit 124 illustrated in Fig. 2 by measuring one or more referential magnetic fields to provide signals indicative of a position of the third exemplary sensor unit 324 and providing an output signal for determining a position of the third exemplary sensor unit 324 within a body.

The inner coil 360 is mounted on a tubular body 28 of the catheter 322. An insulating sleeve 380 is disposed over the inner coil 360. Alternatively, the inner coil 360 can be positioned within a lumen of the tubular body 28. As another alternative, the insulating sleeve 380 can be omitted so that the outer coil 370 is disposed directly over the inner coil 360 and the wire insulation of the inner coil 360 is sufficient to electrically insulate the inner coil 360.

Windings 371 of the outer coil 370 are separated by a distance d1. The winding separation distance d1 can be sufficient to ensure that adjacent uninsulated portions of the outer coil 370 do not touch to result in an electrical short. In some examples, the winding separation distance d1 can be such that a curvature of the outer coil 370 results in a short circuit between windings 371, thereby changing coil impedance as disclosed in greater detail in relation to Figs. 7A and 7B.

The catheter 322 can include one or more sensor units near the distal end 322d of the catheter 322. Fig. 5B illustrates three of the third exemplary sensor units 324 near the distal end 322d of the catheter 322. Alternatively, one, two, four, or five sensor units can be positioned near the distal end 322d of the catheter. Further, the catheter 322 can be modified by replacing some or all of the third exemplary sensor units 324 with any of the other exemplary sensor units 124, 224, 424, 524, 624, 724, 824, 924, 1024, 1124 illustrated herein, the sensor unit 24 including compatible features of any of the exemplary sensor units illustrated herein, variations thereof, or other sensor unit as understood by a person skilled in the pertinent art.

The third exemplary sensor units 324 can each include conductors 372, 374 extending proximally from the outer coil 370 to provide electrical connection from the outer coil to the catheter handle, and thereby to the workstation 55 (Fig. 1). The third exemplary sensor units 324 can each include conductors 337 to provide electrical connection from the inner coil 360 to the catheter handle, and thereby to the workstation 55 (Fig. 1). Likewise, any of the exemplary sensor units disclosed herein can include similar conductors to make electrical connection to the workstation 55 (Fig. 1).

The catheter 322 can further include additional catheter features such as ring electrodes 390, tip electrode 26 (Fig. 1) or other such features as understood by a person skilled the pertinent art. The ring electrodes 390 can function as ACL sensors. The ACL sensor electrodes 26 may provide position sensor measurements that supplement position measurements by the sensor unit 24 (Fig. 1).

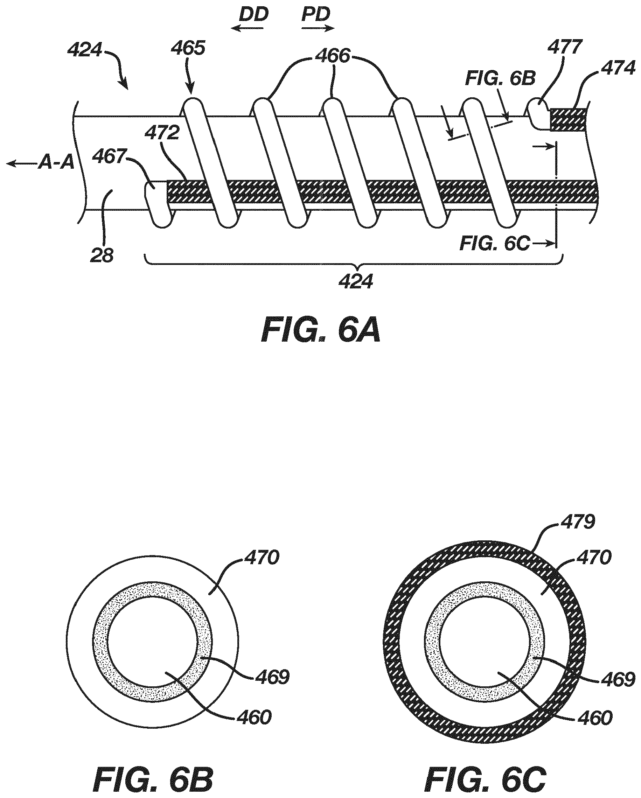

Fig. 6A is an illustration of a fourth exemplary sensor unit 424 illustrating features of the sensor unit 24 of Fig. 1. A coaxial wire 465 is wound around a longitudinal axis A-A to form the fourth exemplary sensor unit 424. The coaxial wire 465 includes windings 466 forming a coil and two insulated return lines 472, 474. The coaxial wire 465 forms a proximal bend 477 between a proximal end of the windings 466 and a proximal return line 474. The coaxial wire 465 forms a distal bend 467 between a distal end of the windings 466 and a distal return line 472. The distal return line 472 is positioned under the windings 466 so that the distal return line 472 does not inhibit the windings 466 from making contact with tissue and/or the external environment.

Figs. 6B and 6C are illustrations of cross-sections of a coaxial wire 465 of the fourth exemplary sensor unit 424 as indicated in Fig. 6A. The coaxial wire 465 includes a core conductive member 460, an inner insulation 469, an outer conductive member 470, and an outer insulation 479. The inner insulation 469 is over the core conductive member 460 and electrically insulating the core conductive member 460 from an external environment to the fourth exemplary sensor unit 424. The outer conductive member extends over the inner insulation and is electrically insulated from the core conductive member 460 by the inner insulation 469. The outer insulation 479 is over a portion of the outer conductive member 470 such that uninsulated portions of the outer conductive member 470 are exposed to the external environment to the fourth exemplary sensor unit 424 at two or more windings 466 of the coaxial wire 465 forming the fourth exemplary sensor unit 424. The core conductive member 460, inner insulation 469, outer conductive member 470, and outer insulation 479 are coaxial to each other and forming the coaxial wire 465, which is wound about the longitudinal axis A-A.

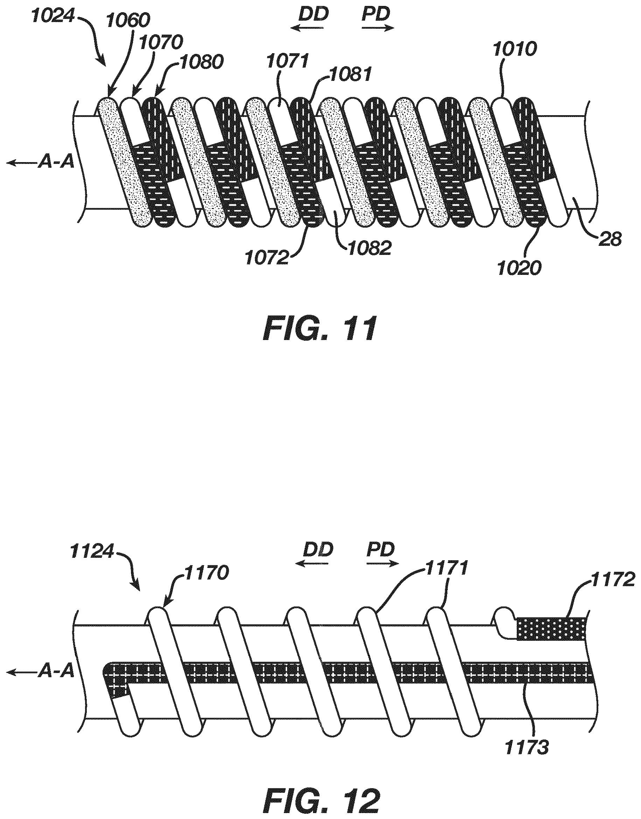

As illustrated, all of the windings 466 of the coil of the coaxial wire 465 are uninsulated. Alternatively, selected portions of the windings 466 may be insulated, for instance, to make ECG and/or environmental measurements that are directionally dependent such as illustrated in greater detail in Fig. 11, or to provide a desired secondary component to the coil impedance in environmental conditions encountered during treatment.

The core conductive member 460 can configured to measure one or more referential magnetic fields to provide signals indicative of a position of the third exemplary sensor unit 324 and output signals for determining a position of the sensor within a body. The core conductive member 460 can function similarly to the inner coil 60 illustrated in Fig. 2 or the inner coil 360 illustrated in Fig. 5A. The core conductive member 460 can be configured output signals based on a change in magnetic flux through the fourth exemplary sensor unit 424.

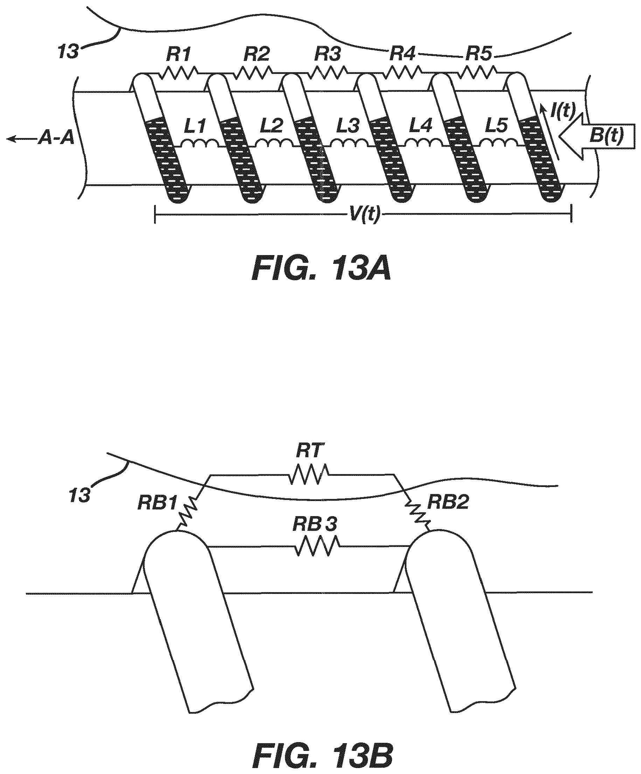

The uninsulated portions of the outer conductive member 470 can be configured to contact tissue and output physiologic signals (e.g, ECG) through the tissue. The outer conductive member 470 can be configured to vary in impedance in response to changes in conductivity of the external environment to which the uninsulated portions are exposed. The change in impedance can be similar to the change in impedance of the outer coil 70 illustrated in Fig. 1, the coil 270 illustrated in Fig. 4, the outer coil 370 illustrated in Figs. 5A and 5B, as disclosed in relation to Figs. 13A and 13B, or as otherwise understood by a person skilled in the art having benefit of the present disclosure. The outer conductive member 470 can be configured to provide output signals indicative of changes in conductivity of the external environment to which the uninsulated portions are exposed. The external environment can cause a change in impedance to the outer conductive member 470, and the impedance can be used by the workstation 55 (Fig. 1) to identify changes in conductivity of the external environment.

The outer conductive member 470 can be configured to measure one or more referential magnetic fields to provide signals indicative of a position of the third exemplary sensor unit 324 and output signals for determining a position of the sensor within a body. For instance, if the secondary impedance between uninsulated wire portions is sufficiently high so that the coil impedance is approximately equal to the inductive portion of the coil impedance of the outer conductive member 470, the electrical signal induced by the magnetic field generated by the magnetic field generators 32 (Fig. 1) can provide information as to the location of the windings 466 within the generated field, and therefore a position within the patient 23. The outer conductive member 470 can be configured output signals based on a change in magnetic flux through the fourth exemplary sensor unit 424. Based on the detected outer conductive member 470, the position of the windings 466 of the fourth exemplary sensor unit 424 in the body, for example, the heart 12, is determined. When the outer conductive member 470 is used as a position sensor, the core conductive member 460 can serve as a reference.

Adjacent windings 466 with uninsulated portions of the outer conductive member 470 can be positioned such that the uninsulated portions are separated by a gap when the sensor is aligned with a straight longitudinal axis. Bending the sensor can causes the gap to collapse to electrically short the uninsulated portions of the adjacent windings 466 to thereby provide an indication of degree of curvature of the sensor as disclosed in greater detail in relation to Figs. 7A and 7B.

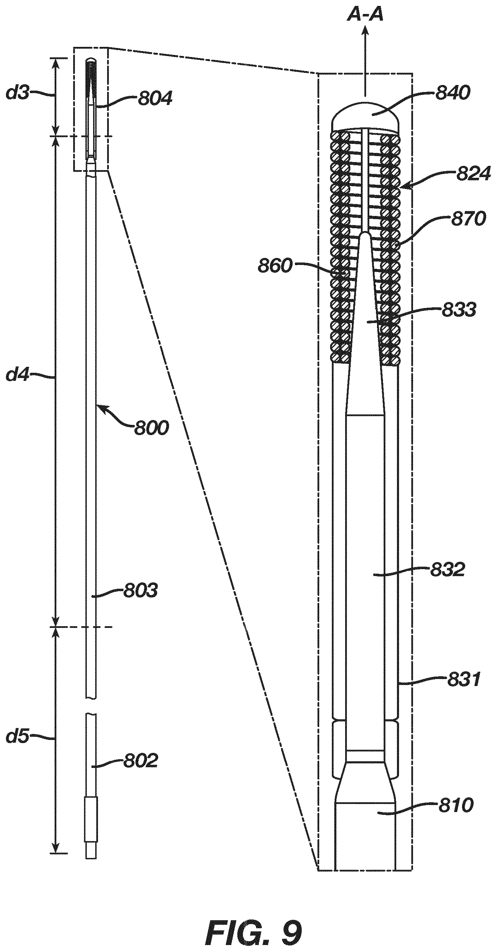

The fourth exemplary sensor unit 424 can be integrated with a medical probe such as a catheter (e.g. see Figs. 1 and 5B) or a guide wire (e.g. Fig. 9). An example catheter can include a tubular shaft extending along the longitudinal axis A-A and the coaxial wire 465 wound around the tubular shaft to form the fourth exemplary sensor unit 242 on the catheter body. An example guide wire can include a tubular shaft extending along the longitudinal axis A-A and the coaxial wire 465 extending from a distal end of the tubular shaft and wound about the longitudinal axis A-A.

Figs. 7A and 7B are illustrations of a fifth exemplary sensor unit 524 illustrating features of the sensor unit 24 of Fig. 1, which can be combined with features of other sensor units disclosed herein, variations thereof, and alternatives thereto as understood by a person skilled in the pertinent art with the benefit of the present disclosure. Fig. 7A is an illustration of the fifth exemplary sensor unit 524 with a linear alignment. Adjacent windings 571 of the coil 570 are separated by a gap distance d1 which is sufficient to electrically insulate the adjacent windings from each other.

Fig. 7B is an illustration of the fifth exemplary sensor unit 524 bent about a radius of curvature r1, which causes three of the illustrated adjacent windings 571 to come into contact with each other at a short location 572 and the gap distance d2 between adjacent windings 571 to increase opposite the short location 572. When the adjacent windings 571 are shorted at the short location 572, one or more winding loops effectively have zero impedance because they are being bypassed by the short. This changes the overall impedance of the coil 570. Change in impedance of the coil can be measured using techniques disclosed in greater detail elsewhere herein, for instance in relation to Figs. 2, 13A, and 13B. The measured change in impedance in the coil is directly dependent on how many windings 571 are bypassed by shorting. The coil geometry, for instance gap distance d1, can be configured such that the number of coils that are shorted varies with the radius of curvature r1 of the fifth exemplary sensor unit 524. Generally, a tighter curve may cause more coils to be shorted. The gap distance d1 may vary along the length of the coil 570 to further manipulate the relationship between radius of curvature r1 and coil impedance.

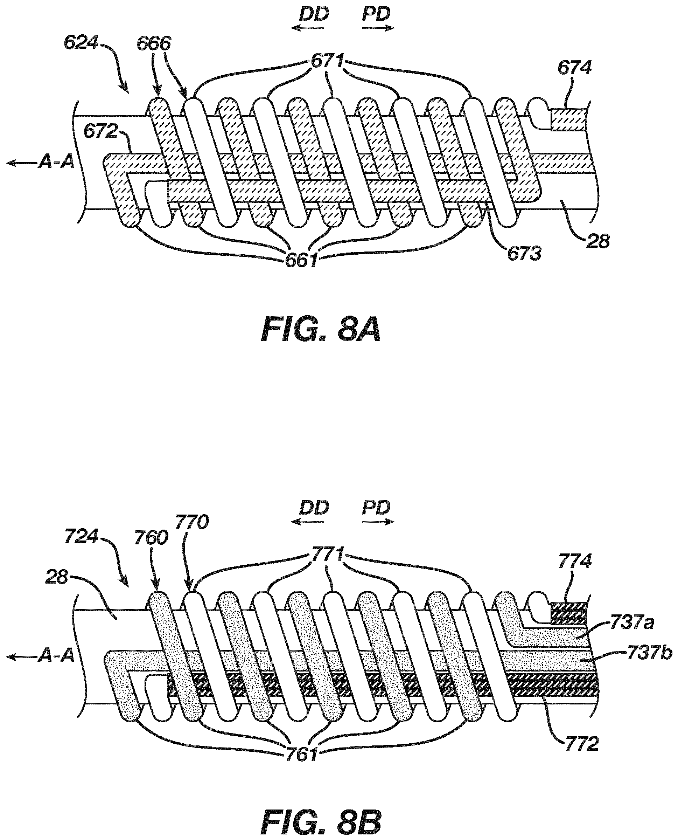

Fig. 8A is an illustration of a sixth exemplary sensor unit 624 illustrating features of the sensor unit 24 of Fig. 1. 13. The sixth exemplary sensor unit 624 includes a first plurality of coil windings 661, a second plurality of coil windings 671, a distal return line 672, a proximal return line 674, and a connecting line 673. The first plurality of coil windings 661 are electrically isolated from an external environment to the sixth exemplary sensor unit 624 and have an inductive impedance independent of conductivity of the external environment. The second plurality of coil windings 671 are interleaved with the first plurality of windings 661 and include at least two windings each at least partially uninsulated and exposed to the external environment of the sixth exemplary sensor unit 624 such that the second plurality of coil windings 671 has an impedance that varies with changes in conductivity of the external environment. The return lines 672, 674 provide electrical connection from the first and second plurality of windings 661, 671 to a catheter handle, and thereby the workstation 55 (Fig. 1). The connecting line 673 joins a distal end of the second plurality of coil windings 671 to a proximal end of the first plurality of coil windings 661.

The first plurality of coil windings 661 of the sixth exemplary sensor unit 624 are electrically in series with the second plurality of coil windings 671. The first plurality of coil windings 661 and the second plurality of coil windings 671 of the sixth exemplary sensor unit 624 are formed from a single wire 666. The single wire 666 can be configured to output signals for determining position of the sensor within a body, determining electrocardiogram signals, and determining proximity to tissue.

The single wire 666 is formed such that the distal return line 672 is positioned first on the tubular body 28, followed by the first plurality of windings 661, followed by the connecting line 673, followed by the second plurality of windings 671, and finally the proximal return line 674. The distal return line 672 has an insulated first length extending distally under the first plurality of coil windings 661 and the second plurality of coil windings 671. An insulated second length of the first plurality of windings 661 coils proximally from the insulated first length of the distal return line 672. An insulated third length of the connecting line 673 extends distally from the insulated second length over the first plurality of coil windings 661. An uninsulated fourth length coils proximally from the insulated third length to form the second plurality of coil windings 671. An insulated fifth length of the proximal return line 674 extends proximally from the second plurality of coil windings 671.

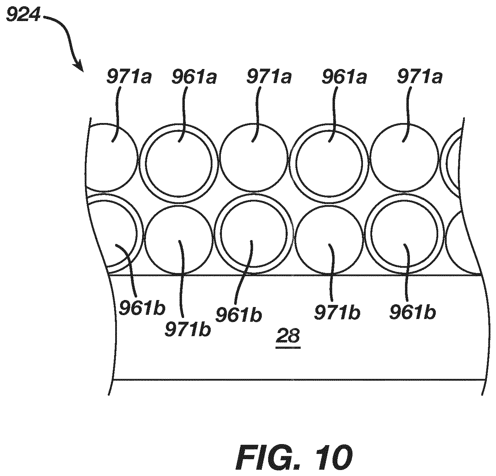

Although not illustrated in Fig. 8A, the first plurality of coil windings 661 can be interleaved with the second plurality of coil windings 671 in two or more layers such that an inner layer of the two or more layers includes uninsulated portions of the second plurality of coil windings 671 electrically isolated from the external environment by an outer layer of the two or more layers. The two or more layers can have a cross-section having a checkerboard pattern with alternating insulated wire portions and uninsulated wire portions. The two or more layers can be configured similar to as disclosed in relation to Fig. 10.

Although not illustrated in Fig. 8A, the second plurality of coil windings 671 can have exposed portions on only a first side of the medical probe such that the impedance of the second plurality of coil windings 671 varies with changes in conductivity of the external environment in the vicinity of the first side of the medical probe. The second plurality of coil windings 671 can be configured similarly to the directional coil windings 1070, 1080 illustrated in Fig. 11.

Fig. 8B is an illustration of a seventh exemplary sensor unit 724 illustrating features of the sensor unit 24 of Fig. 1. The seventh exemplary sensor unit 724 includes a first plurality of coil windings 761 and a second plurality of coil windings 771. The first plurality of coil windings 761 are electrically isolated from an external environment to the seventh exemplary sensor unit 724 and has an inductive impedance independent of conductivity of the external environment. The second plurality of coil windings 771 are interleaved with the first plurality of windings 761. The second plurality of coil windings 771 include at least two windings each that are at least partially uninsulated and exposed to the external environment of the seventh exemplary sensor unit 724 such that the second plurality of coil windings 771 has an impedance that varies with changes in conductivity of the external environment.

The seventh exemplary sensor unit 724 can be configured to output signals for determining position of the sensor within a body, determining electrocardiogram signals, and determining proximity to tissue.

The first plurality of coil windings 761 are electrically isolated from the second plurality of coil windings 771.

The seventh exemplary sensor unit 724 further includes a first wire 760 and a second wire 770. The first wire 760 includes the first plurality of windings 761. The first wire 760 is configured to measure one or more referential magnetic fields to provide signals indicative of a position of the third exemplary sensor unit 324 and output signals for determining a position of the seventh exemplary sensor unit 724 within a body. The second wire 770 includes the second plurality of windings 771 and is configured to output electrocardiogram signals and signals indicative of a change in electrical conductivity of the external environment.

Although not illustrated in Fig. 8B, the first plurality of coil windings 761 can be interleaved with the second plurality of coil windings 771 in two or more layers such that an inner layer of the two or more layers includes uninsulated portions of the second plurality of coil windings 771 electrically isolated from the external environment by an outer layer of the two or more layers. The two or more layers can have a cross-section having a checkerboard pattern with alternating insulated wire portions and uninsulated wire portions. The two or more layers can be configured similar to as disclosed in relation to Fig. 10.

Although not illustrated in Fig. 8B, the second plurality of coil windings 771 can have exposed portions on only a first side of the medical probe such that the impedance of the second plurality of coil windings 771 varies with changes in conductivity of the external environment in the vicinity of the first side of the medical probe. The second plurality of coil windings 771 can be configured similarly to the directional coil windings 1070, 1080 illustrated in Fig. 11.

Fig. 9 is an illustration of a guide wire 800 including an eighth exemplary sensor unit 824. The guidewire has a proximal portion 802 configured to be manipulated outside the patient, a mid-portion configured to extend through vasculature, and a distal end 804 configured to navigate vasculature. The guide wire 800 includes a tubular shaft 810 extending along a longitudinal axis A-A and the eighth exemplary sensor unit 824 extending from a distal end of the tubular shaft. The distal end of the tubular shaft can include a tapered inner portion 832 and a tubular portion 831 over the tapered inner portion 832. The eighth exemplary sensor unit 824 includes an inner coil 860 over a tapered end 833 of the tapered inner portion 832, an insulative jacket 850 over the inner coil 860, and an outer coil 870 over the insulative jacket 850 the guide wire 800 includes an atraumatic end cap 840 at a distal end of the guide wire 800. The eighth exemplary sensor unit 824 can alternatively be configured similar other wrapped-wire exemplary sensor units 124, 224, 324, 424, 524, 624, 724, 924, 1024, 1124 illustrated herein, variations thereof, and alternatives thereto as understood by a person skilled in the pertinent art.

Fig. 10 s an illustration of a ninth exemplary sensor unit 924 illustrating features of the sensor unit 24 of Fig. 1. The ninth exemplary sensor unit 924 includes a plurality of insulated windings 961a, 961b and a plurality of uninsulated windings 971a, 971b interleaved with the insulated windings 961a, 961b in two or more layers. An inner layer of the two or more layers includes uninsulated portions of coil windings 971b electrically isolated from the external environment by an outer layer of the two or more layers. The plurality of insulated windings 961a, 961b are interleaved with a plurality of uninsulated windings 971a, 971b in two or more layers with a cross-section having a checkerboard pattern with alternating insulated wire 961a, 961b portions and uninsulated wire portions 971a, 971b.

Fig. 11 is an illustration of a tenth exemplary sensor unit 1024 illustrating features of the sensor unit 24 of Fig. 1. The tenth exemplary sensor unit 1024 includes a first plurality of coil windings 1060, a second plurality of coil windings 1070, and a third plurality of coil windings 1080. The first plurality of coil windings 1060 are electrically isolated from an external environment to the tenth exemplary sensor unit 1024 and includes an inductive impedance independent of conductivity of the external environment. The second plurality of coil windings 1070 includes at least two windings each at least partially uninsulated and exposed to the external environment on a first side 1010 of the tenth exemplary sensor unit 1024 such that the second plurality of coil windings 1070 has an impedance that varies with changes in conductivity of the external environment on the first side 1010. The third plurality of coil windings 1080 includes at least two windings each at least partially uninsulated and exposed to the external environment on a second side 1020 of the tenth exemplary sensor unit 1024 such that the third plurality of coil windings 1080 has an impedance that varies with changes in conductivity of the external environment on the second side 1020. The first plurality of coil windings 1060, the second plurality of coil windings 1070, and the third plurality of coil windings 1080 are interleaved with each other along a surface of a tubular support 28 and wound about a longitudinal axis A-A.

The tenth exemplary sensor unit 1024 can be configured to output signals for determining position of the sensor within a body, determining electrocardiogram signals, and determining proximity to tissue.

The second plurality of coil windings 1070 has exposed portions on only the first side 1010 of the medical probe such that the impedance of the second plurality of coil windings 1070 varies with changes in conductivity of the external environment in the vicinity of the first side 1010 of the medical probe. The third plurality of coil windings 1080 has exposed portions on only the second side 1020 of the medical probe such that the impedance of the third plurality of coil windings 1080 varies with changes in conductivity of the external environment in the vicinity of the second side 1020 of the medical probe. The second plurality of coil windings 1070 and the third plurality of coil windings 1080 are configured to output signals indicative of a direction of tissue) in relation to the first side 1010 and the second side 1020.

Fig. 12 is an illustration of an eleventh exemplary sensor unit 1124 illustrating features of the sensor unit 24 of Fig. 1. The eleventh exemplary sensor unit 1124 includes temperature sensitive conductors that vary in impedance with change in temperature. The eleventh exemplary sensor unit 1124 includes a coil 1170 with windings 1171 about a longitudinal axis A-A, a proximal return line 1172, and a distal return line 1173. The windings 1171 and return lines 1172, 1173 can include materials that differ in temperature sensitivity to each other. The eleventh exemplary sensor unit 1124 has at least two segments that differ in temperature sensitivity.

Figs. 13A and 13B are illustrations of an impedance model of the sensor unit 24 of Fig. 1. As illustrated in Fig. 13A, each winding of the coil has an inductive impedance L1, L2, L3, L4, L5, and the coil has resistive impedance R1, R2, R3, R4, R5 between adjacent exposed coil portions. A varying magnetic field B(t) through the axis A-A of the coil induces a current I(t) through the windings and a voltage V(t) across the coil that can be measured as an output signal for the sensor unit 24. If the varying magnetic field B(t) and the inductive impedance L1, L2, L3, L4, L5 are known, a resistive impedance of the coil can be calculated based on the output signal of the sensor unit 24.

As illustrated in Fig. 13B, the resistive impedance R1, R2, R2, R4, R5 (Fig. 13A) between exposed wire portions, when in vasculature or the heart, is dependent on a path that electrical current can take between adjacent exposed portions of coil windings. Fig. 13B shows two current paths: a first path through blood RB1, through tissue RT, and through blood again RB2; and a second path RB3 directly through blood. When the coil is away from tissue, the second, direct path, has lower resistance than the first path, and the resistive impedance between exposed wire portions is dominated by the direct path RB3 through blood. As the coil is moved near tissue 13, the impedance of the first path becomes comparable to, or less than the second path, and the resistive impedance between exposed wire portions is based on impedance through blood RB1, RB2 and tissue RT. If the varying magnetic field B(t) is applied at a frequency at which the tissue resistance RT is significantly less than that of blood RB1, RB2, the effective resistive impedance of the coil decreases as the coil approaches the tissue 13. Note that the model illustrated in Fig. 13A and 13B is a simplification purely for the sake of illustration. Tissues and/or blood may have a complex impedance. Different tissue types have different impedance, and impedance of blood and tissues vary with frequency.

## Claims

1. A coaxial wire (465) wound around a longitudinal axis to form a sensor for a medical probe, the coaxial wire comprising:
a core conductive member (460);
an inner insulation (469) over the core conductive member and electrically insulating the core conductive member from an external environment to the sensor;
an outer conductive member (470) extending over the inner insulation and electrically insulated from the core conductive member by the inner insulation; and
an outer insulation (479) over a portion of the outer conductive member such that uninsulated portions of the outer conductive member are exposed to the external environment to the sensor at two or more windings (466) of the coaxial wire forming the sensor.

2. The coaxial wire of claim 1, the core conductive member being configured to measure one or more referential magnetic fields to provide signals indicative of a position of the sensor within a body and/or to output signals based on a change in magnetic flux through the sensor.

3. The coaxial wire of any preceding claim, the uninsulated portions being configured to contact tissue and output electrocardiogram (ECG) signals through the tissue.

4. The coaxial wire of any preceding claim, the outer conductive member being configured to vary in impedance in response to changes in conductivity of the external environment to which the uninsulated portions are exposed.

5. The coaxial wire of any preceding claim, the outer conductive member being configured to provide output signals indicative of changes in conductivity of the external environment to which the uninsulated portions are exposed.

6. The coaxial wire of any preceding claim, the outer conductive member being configured to measure one or more referential magnetic fields to provide signals indicative of a position of the sensor within a body.

7. The coaxial wire of any preceding claim, the outer conductive member being configured output signals based on a change in magnetic flux through the sensor.

8. The coaxial wire of any preceding claim, the core conductive member, inner insulation, and outer conductive member being coaxial to each other and wound about the longitudinal axis.

9. The coaxial wire of any preceding claim, comprising adjacent windings with uninsulated portions of the outer conductive member such that the uninsulated portions are separated by a gap when the sensor is aligned with a straight longitudinal axis, and bending the sensor causes the gap to collapse to electrically short the uninsulated portions of the adjacent windings to thereby provide an indication of degree of curvature of the sensor.

10. A catheter (22) comprising:
a tubular shaft (28) extending along a longitudinal axis; and
the coaxial wire of any preceding claim wound around the tubular shaft forming a sensor.

11. A guide wire (800) comprising:
a tubular shaft (810) extending along a longitudinal axis; and
the coaxial wire of any one of claims 1 to 9 extending from a distal end of the tubular shaft and wound about the longitudinal axis forming a sensor.

12. The catheter of claim 10 or the guide wire of claim 11, the core conductive member being configured to measure one or more referential magnetic fields to provide signals indicative of a position of the sensor within a body.

13. The catheter of claim 10 or the guide wire of claim 11, the uninsulated portions being configured to contact tissue and output electrocardiogram (ECG) signals through the tissue.

14. The catheter of claim 10 or the guide wire of claim 11, the outer conductive member being configured to provide output signals indicative of changes in conductivity of the external environment to which the uninsulated portions are exposed.

15. The catheter of claim 10 or the guide wire of claim 11, the core conductive member, inner insulation, and outer conductive member being coaxial to each other and wound about the longitudinal axis.

## Patentansprüche

1. Koaxialdraht (465), der um eine Längsachse gewickelt ist, um einen Sensor für eine medizinische Sonde zu bilden, wobei der Koaxialdraht umfasst:
ein leitfähiges Kernelement (460);
eine innere Isolierung (469) über dem leitfähigen Kernelement, die das leitfähige Kernelement von einer äußeren Umgebung zu dem Sensor elektrisch isoliert;
ein äußeres leitfähiges Element (470), das sich über die innere Isolierung erstreckt und durch die innere Isolierung elektrisch von dem leitfähigen Kernelement isoliert ist; und
eine äußere Isolierung (479) über einem Abschnitt des äußeren leitfähigen Elements, sodass nicht isolierte Abschnitte des äußeren leitfähigen Elements der äußeren Umgebung zu dem Sensor an zwei oder mehr Windungen (466) des Koaxialdrahts, der den Sensor bildet, ausgesetzt sind.

2. Koaxialdraht nach Anspruch 1, wobei das leitfähige Kernelement konfiguriert ist, um ein oder mehrere referenzielle Magnetfelder zu messen, um Signale bereitzustellen, die eine Position des Sensors innerhalb eines Körpers angeben, und/oder um Signale basierend auf einer Änderung des magnetischen Flusses durch den Sensor auszugeben.

3. Koaxialdraht nach einem der vorstehenden Ansprüche, wobei die nicht isolierten Abschnitte konfiguriert sind, um mit Gewebe in Kontakt zu kommen und Elektrokardiogramm-Signale (EKG-Signale) durch das Gewebe auszugeben.

4. Koaxialdraht nach einem der vorstehenden Ansprüche, wobei das äußere leitfähige Element konfiguriert ist, um seine Impedanz als Reaktion auf Änderungen der Leitfähigkeit der äußeren Umgebung zu variieren, der die nicht isolierten Abschnitte ausgesetzt sind.

5. Koaxialdraht nach einem der vorstehenden Ansprüche, wobei das äußere leitfähige Element konfiguriert ist, um Ausgangssignale bereitzustellen, die Änderungen der Leitfähigkeit der äußeren Umgebung angeben, der die nicht isolierten Abschnitte ausgesetzt sind.

6. Koaxialdraht nach einem der vorstehenden Ansprüche, wobei das äußere leitfähige Element konfiguriert ist, um ein oder mehrere referenzielle Magnetfelder zu messen, um Signale bereitzustellen, die eine Position des Sensors innerhalb eines Körpers angeben.

7. Koaxialdraht nach einem der vorstehenden Ansprüche, wobei das äußere leitfähige Element konfiguriert ist, um Signale basierend auf einer Änderung des magnetischen Flusses durch den Sensor auszugeben.

8. Koaxialdraht nach einem der vorstehenden Ansprüche, wobei das leitfähige Kernelement, die innere Isolierung und das äußere leitfähige Element koaxial zueinander und um die Längsachse gewunden sind.

9. Koaxialdraht nach einem der vorstehenden Ansprüche, umfassend benachbarte Windungen mit nicht isolierten Abschnitten des äußeren leitfähigen Elements, sodass die nicht isolierten Abschnitte durch einen Spalt getrennt sind, wenn der Sensor an einer geraden Längsachse ausgerichtet ist, und das Biegen des Sensors bewirkt, dass der Spalt kollabiert, um die nicht isolierten Abschnitte der benachbarten Windungen elektrisch kurzzuschließen, um dadurch eine Angabe des Krümmungsgrads des Sensors bereitzustellen.

10. Katheter (22), umfassend:
einen röhrenförmigen Schaft (28), der sich entlang einer Längsachse erstreckt; und
den Koaxialdraht nach einem der vorstehenden Ansprüche, der um den röhrenförmigen Schaft gewickelt ist und einen Sensor bildet.

11. Führungsdraht (800), umfassend:
einen röhrenförmigen Schaft (810), der sich entlang einer Längsachse erstreckt; und
den Koaxialdraht nach einem der Ansprüche 1 bis 9, der sich von einem distalen Ende des röhrenförmigen Schafts erstreckt und um die Längsachse gewunden ist und einen Sensor bildet.

12. Katheter nach Anspruch 10 oder Führungsdraht nach Anspruch 11, wobei das leitfähige Kernelement konfiguriert ist, um ein oder mehrere referenzielle Magnetfelder zu messen, um Signale bereitzustellen, die eine Position des Sensors innerhalb eines Körpers angeben.

13. Katheter nach Anspruch 10 oder Führungsdraht nach Anspruch 11, wobei die nicht isolierten Abschnitte konfiguriert sind, um mit Gewebe in Kontakt zu kommen und Elektrokardiogramm-Signale (EKG-Signale) durch das Gewebe auszugeben.

14. Katheter nach Anspruch 10 oder Führungsdraht nach Anspruch 11, wobei das äußere leitfähige Element konfiguriert ist, um Ausgangssignale bereitzustellen, die Änderungen der Leitfähigkeit der äußeren Umgebung angeben, der die nicht isolierten Abschnitte ausgesetzt sind.

15. Katheter nach Anspruch 10 oder Führungsdraht nach Anspruch 11, wobei das leitfähige Kernelement, die innere Isolierung und das äußere leitfähige Element koaxial zueinander und um die Längsachse gewunden sind.

## Revendications

1. Fil coaxial (465) enroulé autour d'un axe longitudinal pour former un capteur pour une sonde médicale, le fil coaxial comprenant :
un élément conducteur central (460) ;
une isolation interne (469) sur l'élément conducteur central et isolant électriquement l'élément conducteur central d'un environnement externe au capteur ;
un élément conducteur externe (470) s'étendant sur l'isolation interne et isolé électriquement de l'élément conducteur central par l'isolation interne ; et
une isolation externe (479) sur une partie de l'élément conducteur externe de telle sorte que des parties non isolées de l'élément conducteur externe sont exposées à l'environnement externe au capteur au niveau de deux enroulements (466) ou plus du fil coaxial formant le capteur.

2. Fil coaxial selon la revendication 1, l'élément conducteur central étant configuré pour mesurer un ou plusieurs champs magnétiques de référence afin de fournir des signaux indiquant une position du capteur à l'intérieur d'un corps et/ou de délivrer en sortie des signaux basés sur un changement de flux magnétique à travers le capteur.

3. Fil coaxial selon l'une quelconque revendication précédente, les parties non isolées étant configurées pour entrer en contact avec le tissu et délivrer en sortie des signaux d'électrocardiogramme (ECG) à travers le tissu.

4. Fil coaxial selon l'une quelconque revendication précédente, l'élément conducteur externe étant configuré pour varier en impédance en réponse à des changements de conductivité de l'environnement externe auquel les parties non isolées sont exposées.

5. Fil coaxial selon l'une quelconque revendication précédente, l'élément conducteur externe étant configuré pour fournir des signaux de sortie indiquant les changements de conductivité de l'environnement externe auquel les parties non isolées sont exposées.

6. Fil coaxial selon l'une quelconque revendication précédente, l'élément conducteur externe étant configuré pour mesurer un ou plusieurs champs magnétiques de référence afin de fournir des signaux indiquant une position du capteur à l'intérieur d'un corps.

7. Fil coaxial selon l'une quelconque revendication précédente, l'élément conducteur externe étant configuré pour délivrer en sortie des signaux basés sur un changement de flux magnétique à travers le capteur.

8. Fil coaxial selon l'une quelconque revendication précédente, l'élément conducteur central, l'isolation interne et l'élément conducteur externe étant coaxiaux les uns par rapport aux autres et enroulés autour de l'axe longitudinal.

9. Fil coaxial selon l'une quelconque revendication précédente, comprenant des enroulements adjacents avec des parties non isolées de l'élément conducteur externe de telle sorte que les parties non isolées sont séparées par un espace lorsque le capteur est aligné sur un axe longitudinal droit, et la flexion du capteur provoque l'affaissement de l'espace pour court-circuiter électriquement les parties non isolées des enroulements adjacents pour fournir ainsi une indication du degré de courbure du capteur.

10. Cathéter (22) comprenant :
une tige tubulaire (28) s'étendant le long d'un axe longitudinal ; et
le fil coaxial selon l'une quelconque revendication précédente enroulé autour de la tige tubulaire formant un capteur.

11. Fil-guide (800), comprenant :
une tige tubulaire (810) s'étendant le long d'un axe longitudinal ; et
le fil coaxial selon l'une quelconque des revendications 1 à 9 s'étendant depuis une extrémité distale de la tige tubulaire et enroulé autour de l'axe longitudinal formant un capteur.

12. Cathéter selon la revendication 10 ou fil-guide selon la revendication 11, l'élément conducteur central étant configuré pour mesurer un ou plusieurs champs magnétiques de référence afin de fournir des signaux indiquant une position du capteur à l'intérieur d'un corps.

13. Cathéter selon la revendication 10 ou fil-guide selon la revendication 11, les parties non isolées étant configurées pour entrer en contact avec le tissu et délivrer en sortie des signaux d'électrocardiogramme (ECG) à travers le tissu.

14. Cathéter selon la revendication 10 ou fil-guide selon la revendication 11, l'élément conducteur externe étant configuré pour fournir des signaux de sortie indiquant des changements de conductivité de l'environnement externe auquel les parties non isolées sont exposées.

15. Cathéter selon la revendication 10 ou fil-guide selon la revendication 11, l'élément conducteur central, l'isolation interne et l'élément conducteur externe étant coaxiaux les uns par rapport aux autres et enroulés autour de l'axe longitudinal.
